# EUROPEAN PATENT APPLICATION

(11) **EP 3 287 136 A1**
(43) Date of publication of application: **28.02.2018**
(21) Application number: 17184913.6
(22) Date of filing: 04.08.2017
(51) Int. Cl.: A61K 36/28, A61P 29/00, A61P 39/00

(54) **COMPOSITION FOR PREVENTING OR TREATING CELL DAMAGE INCLUDING YOUNGIA DENTICULATA EXTRACT**

(30) Priority: 18.08.2016 KR 20160104925; 31.07.2017 KR 20170096844
(71) Applicant: Incospharm Corporation, Yuseong-gu Daejeon 34055 (KR)
(72) Inventor: Lim, Chae Jin, Yuseong-gu Daejeon, 34198 (KR); Yoon, Seok Jeong, Daejeon, 35210 (KR); Kor, Myung Ho, Daejeon, 34116 (KR); Chung, Hwa-Jee, Daejeon, 34074 (KR); Jung, Ju Yeon, Sejong-si, 30100 (KR); Shin, Ka Young, Daejeon, 35210 (KR); Park, Kee Don, Daejeon, 34890 (KR)
(74) Representative: Adam, Holger

(57) **Abstract**

Provided is *Youngia denticulata* extract being effective for prevention and treatment of diseases caused by an environmental pollution substance, and capable of protecting cell damage.

## Description

### CROSS-REFERENCE TO RELATED APPLICATIONS

This application claims priority to Korean Patent Application No. 10-2016-0104925, filed on 08. 18. 2016 and Application No. 10-2017-0096844, filed on 07. 31. 2017, in the Korean Intellectual Property Office, the disclosure of which is incorporated herein by reference in its entirety.

### TECHNICAL FIELD

The following disclosure relates to a composition including *Youngia denticulata* extract capable of preventing or treating cell damage or generic material damage by an environmental pollution substance.

### BACKGROUND

As the environmental pollution gets worse, various diseases appear in the whole organisms including a human being by environmental pollution substances. There are many kinds of environmental pollution substances, and it is currently almost impossible to treat or prevent all of various diseases or negative phenomenon in an organism which may be induced by the environmental pollution substance.

As the representative of the environmental pollution substance, polycyclic aromatic hydrocarbon (PAH) and heavy metals are well known in the art. The polycyclic aromatic hydrocarbon is aromatic hydrocarbon having several benzene rings, and may be produced by various activities such as smoking or a cooking process as well as industrial activities. The polycyclic aromatic hydrocarbon may be a carcinogen or a mutagen even with a trace amount, and may be a main neotoxin leading to malfunction of the normal organ of an organism as well as teratogenesis. For example, benzo-[a]-pyrene, representative of the polycyclic aromatic hydrocarbon is included in the polluted atmosphere, and introduced into the living body through breathing, or also through burnt food intake, smoking or the like, thereby causing cytotoxicity of normal cells, or modifying or damaging genetic material in normal cells.

Further, the heavy metals may be absorbed in the body only by breathing and skin contact, and cause various addiction symptoms and diseases. Once the heavy metals are absorbed in the body, it is not released and accumulated therein, and thus, it is difficult to treat the disease caused by the heavy metals, and very fatal diseases such as malformation and lung cancer are caused, and thus, the heavy metals are very dangerous.

It is needed to develop a method or material for solving these problems caused by the environmental pollution substance. Particularly, a method of using natural extract or a natural organism which is very likely to be harmless to an organism may be better. For example, Korean Patent Laid-Open Publication No. 10-1999-0046464 discloses a method of treating wastewater and an air pollution substance using earthworms.

### [Related Art Document]

### [Patent Document]

Korean Patent Laid-Open Publication No. 10-1999-0046464

### SUMMARY

An embodiment of the present invention is directed to providing prevention and treatment of various damage by a polycyclic aromatic hydrocarbon (PAH)-based environmental pollution substance and heavy metals, using *Youngia denticulata* extract as an anti-environmental pollution substance.

In one general aspect, a health functional food for prevention or treatment of diseases caused by a polycyclic aromatic hydrocarbon-based environmental pollution substance includes *Youngia denticulata* extract as an effective component.

In another general aspect, an anti-inflammatory health functional food against inflammation caused by a polycyclic aromatic hydrocarbon-based environmental pollution substance includes *Youngia denticulata* extract as an effective component.

In another general aspect, an anti-inflammatory cosmetic composition against inflammation caused by a polycyclic aromatic hydrocarbon-based environmental pollution substance includes *Youngia denticulata* extract as an effective component.

In another general aspect, a composition for prevention or treatment of cell damage caused by a, polycyclic aromatic hydrocarbon-based environmental pollution substance includes *Youngia denticulata* extract as an effective component.

In another general aspect, a composition for prevention or treatment of cell damage caused by heavy metals includes *Youngia denticulata* extract as an effective component.

In another general aspect, a food composition for releasing heavy metals includes *Youngia denticulata* extract as an effective component.

Other features and aspects will be apparent from the following detailed description, the drawings, and the claims.

### BRIEF DESCRIPTION OF THE DRAWINGS

FIG. 1 represents cell viability when treating cytotoxicity-induced cells by treatment with benzo-[a]-pyrene(BaP) among polycyclic aromatic hydrocarbon-based environmental pollution substances with *Youngia denticulata* extract.
FIGS. 2A to 2C represent cell viability when treating cytotoxicity-induced cells by treatment with cadmium (A), lead (B) and nickel (C) among heavy metal pollution substances having harmful toxicity with *Youngia denticulata* extract.
FIG. 3 represents a degree of reducing inflammation inducing gene expression when treating inflammatory factors increased by treatment with benzo-[a]-pyrene(BaP) among polycyclic aromatic hydrocarbon-based environmental pollution substances with *Youngia denticulata* extract.

### DETAILED DESCRIPTION OF EMBODIMENTS

The advantages, features and aspects of the present invention will become apparent from the following description of the embodiments with reference to the accompanying drawings, which is set forth hereinafter. The present invention may, however, be embodied in different forms and should not be construed as limited to the embodiments set forth herein. Rather, these embodiments are provided so that this disclosure will be thorough and complete, and will fully convey the scope of the present invention to those skilled in the art. The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Hereinafter, exemplary embodiments will be described in detail with reference to the accompanying drawings.

*Youngia denticulata* which is a plant of Anthophyta Monosepalous Campanulales Asteraceae, also referred to as *Crepidiastrum denticulatum,* is an annual or biennial plant. It is a plant that grows mainly in dry areas and can be easily harvested from mountains, fields of Korea, Japan, and China.

The *Youngia denticulata* extract of the present invention is a natural material, and may be used as an anti-environmental pollution substance. For example, it may prevent or treat diseases and cell damage caused by a polycyclic aromatic hydrocarbon (PAH)-based environmental pollution substance and heavy metals.

Specifically, according to the present invention, a health functional food including the *Youngia denticulata* extract as an effective component may be used for prevention or treatment of the diseases caused by a polycyclic aromatic hydrocarbon-based environmental pollution substance.

According to the present invention, a health functional food including the *Youngia denticulata* extract as an effective component may be used as an anti-inflammatory food against inflammation caused by a polycyclic aromatic hydrocarbon-based environmental pollution substance.

According to the present invention, a cosmetic composition including the *Youngia denticulata* extract as an effective component may be used as an anti-inflammatory composition against inflammation caused by a polycyclic aromatic hydrocarbon-based environmental pollution substance.

According to the present invention, a pharmaceutical composition including the *Youngia denticulata* extract as an effective component may be used as a composition for prevention or treatment of cell damage caused by a polycyclic aromatic hydrocarbon-based environmental pollution substance.

According to the present invention, a health functional food including the *Youngia denticulata* extract as an effective component may be used for prevention or treatment of cell damage caused by a polycyclic aromatic hydrocarbon-based environmental pollution substance.

According to the present invention, a health functional food including the *Youngia denticulata* extract as an effective component may be used for prevention or treatment of cell damage caused by heavy metals.

According to the present invention, a food composition including the *Youngia denticulata* extract as an effective component may be used for releasing heavy metals.

In addition, a composition including the *Youngia denticulata* extract as an effective component may be used for neutralizing or removing a polycyclic aromatic hydrocarbon-based environmental pollution substance and heavy metals. For example, it may be used as a skin wash, a wash for cleaning, a composition for removing fine dust, a composition for skin care, a pollution substance remover, and the like.

When almost all of the compounds included in the polycyclic aromatic hydrocarbon-based environmental pollution substance are exposed, cytotoxicity is induced to result in apoptosis. Further, those compounds may directly damage the genetic material of cells to rapidly suppress normal cell activities such as normal cell respiration, division or differentiation.

In particular, benzo-[a]-pyrene representative of the polycyclic aromatic hydrocarbon-based environmental pollution substances may induce apoptosis caused by cytotoxicity, or abnormal cell activity or apoptosis by damaged genetic material such as DNA. Further, benzo-[a]-pyrene may cause various immune diseases, and reduce production of testosterone and sperm in a testis, a male genital organ. In addition, benzo-[a]-pyrene is introduced and accumulated in body cells through breathing, food intake, skin and the like to cause inflammation, inflammatory diseases, skin cancer, lung cancer, emphysema, colon cancer, chronic cough and chronic bronchitis.

Specifically, as the diseases caused by benzo-[a]-pyrene in the body, lung cancer, emphysema, colon cancer, rash, chronic cough, chronic bronchitis, bronchopulmonary cancer, warts, erythema, anemia, hyperpigmentation, closure, edema, skin cancer and a behavior disorder, irritation induction to skin and eye, allergic skin reaction, and the like are known from various references.

There are so many kinds of polycyclic aromatic hydrocarbon-based environmental pollution substance, and it may be present in water as well as in the atmosphere. The representative of its kinds is acenaphthene, fluorene, phenanthrene, anthracene, pyrene, benzo-[a]-anthracene, chrysene, benzo-[b]-fluoranthene, benzo-[k]-fluoranthene, benzo-[a]-pyrene, indeno(1,2,3-cd)pyrene, dibenzo-[a,h]-anthracene, benzo(ghi)perylene and the like.

In particular, benzo-[a]-pyrene is well known in the art as a representative polycyclic aromatic hydrocarbon-based environmental pollution substance. Benzo-[a]-pyrene may be absorbed into the body through inhalation or skin, and is oxidized in the body and metabolized into various compounds. Specifically, benzo-[a]-pyrene may be changed to the oxides or metabolites of benzo-[a]-pyrene including enzo-[a]-pyrene-7,8-dihydrodiol, benzo-[a]-pyrene-diol epoxide, benzo-[a]-pyrene dialdehyde, 1,6-benzo-[a]-pyrene quinine, 3,6-benzo-[a]-pyrene quinine, 6,12-benzo-[a]-pyrene quinine, benzo-[a]-pyrene-phenol and their isomers. That is, in the case that benzo-[a]-pyrene may be removed, cell damage and diseases caused by not only benzo-[a]-pyrene but also the oxides and metabolites of benzo-[a]-pyrene may be prevented or treated.

According to an exemplary embodiment of the present invention, *Youngia denticulata* extract may reduce an inflammation response caused by benzo-[a]-pyrene. Further, according to an exemplary embodiment of the present invention, *Youngia denticulata* extract may recover cell damage caused by benzo-[a]-pyrene. These exemplary embodiments show that the *Youngia denticulata* extract may be used as the effective component of a composition for treatment or prevention of the diseases caused by benzo-[a]-pyrene, the oxides of benzo-[a]-pyrene and the metabolites of benzo-[a]-pyrene.

Heavy metals, representative of the environmental pollution substance together with the polycyclic aromatic hydrocarbon-based substance form a very stable organic composite in the body, and block sugar, protein and various energy metabolic systems to cause toxicity. Further, some heavy metals influence a hormone system and are classified as endocrine disruptors having an extensive toxic effect in an endocrine system including a reproductive function. In addition, these heavy metals may cause serious toxicity in later generations, unlike universal acute and chronic addiction. Furthermore, due to their physicochemical properties, they are not easily removed even by self-purification of ecosystem, and thus, the risk thereof is serious.

These polycyclic aromatic hydrocarbon-based environmental pollution substance and heavy metals are harmful components contained a lot in fine dust and ultrafine dust, and when these materials are finely broken down, they may easily enter the body through the respiratory organs and skin. The polycyclic aromatic hydrocarbon-based substance and the heavy metals entering the body irritate various cells such as epithelial cells and lung cells, thereby resulting in a cytokine reaction increasing IL-6, IL-8 and the like to cause inflammation in the body.

According to an exemplary embodiment of the present invention, the *Youngia denticulata* extract may reduce an inflammation response caused by heavy metals. Further, according to another exemplary embodiment of the present invention, the *Youngia denticulata* extract may recover cell damage caused by heavy metals. These exemplary embodiments show that the *Youngia denticulata* extract may be used as the effective component of a composition for treatment or prevention of the diseases caused by heavy metal poisoning. Further, these exemplary embodiments show that the *Youngia denticulata* extract may be used as the effective component of a composition for releasing, removing or detoxifying heavy metals. In addition, fine dust includes heavy metals such as nickel, cadmium and lead, and the *Youngia denticulata* extract may be used for removing or releasing fine dust. Furthermore, the *Youngia denticulata* extract may be used for prevention and treatment of the diseases such as inflammation or aging by fine dust.

In the present invention, the heavy metals are one or more selected from the group consisting of cadmium, lead, arsenic, nickel and mercury, but not limited thereto, and may include the heavy metals being not released from and accumulated in the body and causing the diseases and cell damage.

As the diseases caused by acute or chronic heavy metal poisoning, osteoporosis, jaundice, nerve damage, memory decay, vomiting, abdominal pain, jaundice, anemia, nausea, dyspnea, anxiety, malaise, kidney disorder, immune depression, deformity birth, infertility, miscarriage, stillbirth, atopy, Minamata disease, itai disease, emphysema, diabetes, cancer, and the like are known from various references.

In the preparation of the *Youngia denticulata* extract in the present invention, an extraction method using alcohol may be used, but not limited thereto, and any extraction method commonly used in the art to which the present invention pertains may be used. In the extraction, it is preferred to use the top parts of *Youngia denticulata* including leaves, flowers, stems and the like, but not limited thereto, and the entire *Youngia denticulata* may be used. As the kind of alcohol, ethanol is preferred, but not limited thereto, and methanol, isopropanol, propanol, butanol and the like may be used. According to an exemplary embodiment of the present invention, the *Youngia denticulata* extract may be prepared by subjecting *Youngia denticulate* to extraction with alcohol, sonication, and filtering, and then collecting the supernatant which was then subjected to concentration under reduced pressure and lyophilization.

The *Youngia denticulata* extract of the present invention may be provided as a pharmaceutical composition including the extract.

When it is provided as a pharmaceutical composition, it may further include an excipient, a diluent or a carrier. For example, sugars such as sucrose, sorbitol, mannitol, xylitol, lactose, dextrose, or water, starch, edible rubber, agar, calcium phosphate, oil, calcium phosphate, talc and the like may be included, but not limited thereto. The pharmaceutical composition may be formulated into various forms known in the medicine fields, depending on various classified administration methods such as oral/parental administration and provided, and is not particularly limited. The dosage may be properly adjusted depending on the body condition of various animals including a human being, and is not particularly limited.

The *Youngia denticulata* extract of the present invention may be provided as a health functional food including the extract.

The health functional food may freely have the forms such as solid/liquid. As the form, it is preferred to use beverage, pills, tablets, powder or capsule. Further, various forms of food additives may be included together in the health functional food and prepared. As a specific example of the functional food, meat, sausage, bread, chocolate, candy, snacks, confections, pizza, ramen, other noodles, gum, dairy products including ice cream, various soups, beverages, tea, drinks, alcoholic beverages and vitamins and the like may be listed.

The *Youngia denticulata* extract of the present invention may be provided as a cosmetic composition including the extract. The cosmetic composition may be provided in the form of solution, emulsion, viscous mixture and the like. The component included in the cosmetic composition may include the components commonly used in the cosmetic composition as an effective component, in addition to the *Youngia denticulata* extract. For example, common adjuvants and carriers such as stabilizers, solubilizers, vitamin, pigment and flavoring may be included. Further, the cosmetic composition may be prepared into any formulation commonly prepared in the art. For example, emulsion, cream, foundation, lotion, a cosmetic liquid, hair cosmetics and the like may be listed. Specifically, the cosmetic composition of the present invention may include a formulation of skin lotion, skin softener, moisturizing lotion, nutrition lotion, massage cream, nutritional cream, hand cream, moisturizing cream, essence, pack, soap, tonic, milk lotion, gel, ointment, patch, cleansing foam, body cleanser, astringent or spray.

Hereinafter, the Examples for carrying out the present invention will be described. The Examples correspond to one illustration for carrying out the present invention, and the present invention is not interpreted as being limited by the Examples.

### [Example 1]

### Preparation of Youngia denticulata extract

100 g of dried *Youngia denticulate* (The Province Of Gyeong Sangbukdo, Korea) was prepared, and divided into 50 g of each, which was placed in a different empty bottle from each other, and then 1000 ml of 70% (w/w) ethanol was added thereto, respectively. After adding ethanol, sonication was carried out for 60 minutes, and filtering was carried out using filter paper. 1000 ml of ethanol was added to the filtrate again, and a process of sonicating for 60 minutes and filtering with filter paper was carried out again two more times to prepare a total of 6000 ml of filtrate. Thereafter, 6000 ml of the filtrate was added to a concentrator, and concentrated to 100 ml at -110 °C. After concentration, it was left in a deep freezer at - 80 °C for 48 hours. Thereafter, 100 ml of the concentrated solution was taken out of the deep freezer and placed in a freeze dryer, and lyophilized under a vacuum condition at - 110 °C for 72 hours. After lyophilization, the *Youngia denticulata* extract was obtained, and used after being stored at -20 °C.

### [Example 2]

### Measurement of effect when treating cytotoxicity induced and genetic material damaged cells with Youngia denticulata extract

It was analyzed whether the *Youngia denticulata* extract of Example 1 provides a significant effect for improvement of cell viability when treating cytotoxicity induced and genetic material damaged cells with the extract.

For analysis, BaP (benzo-[a]-pyrene) as the polycyclic aromatic hydrocarbon-based environmental pollution substance, and cadmium (Cd, cadmium chloride), lead (Pb, lead acetate) and nickel (Ni, nickel chloride) as heavy metals were used.

BaP is a representative polycyclic aromatic hydrocarbon-based environmental pollution substance and the most toxic material among air pollution substances, and causes cell toxicity, genotoxicity, immune diseases, reduced production of testosterone and sperm in a testis, lung cancer, emphysema, colon cancer, chronic cough and chronic bronchitis, and recently, was defined as a main carcinogen.

Cadmium, lead and nickel are representative heavy metals influencing an ecosystem among many heavy metals, and are defined as a specific atmospheric hazardous substances among the priority substance for management included in the master plan of Ministry of Environment of the Republic of Korea, and in the United States, are included in the main environmental pollution substance list, and thus, prevention of introduction into the environmental ecosystem and removal by treatment are emphasized for those metals.

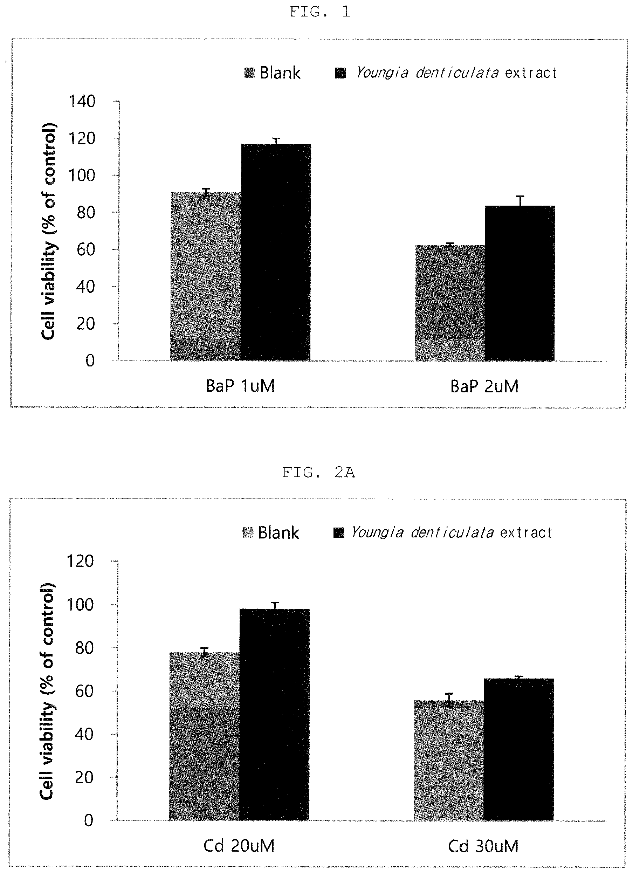

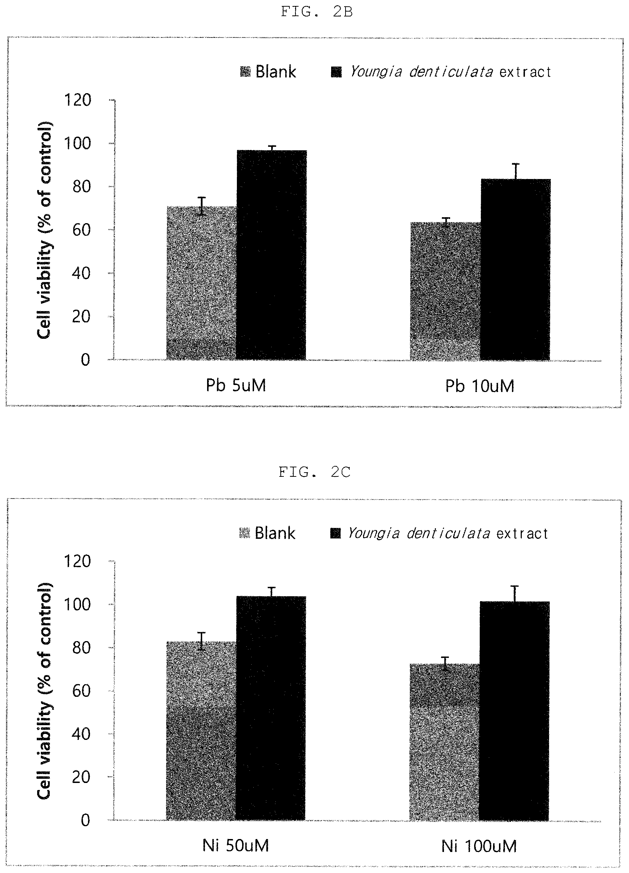

HDFn cells (Human Dermal Fibroblasts, neonatal), human-derived fibroblasts were constantly divided into 6,000 cells on a 96 well plate for culture, and cultured in a Medium 106 (Gibco Cascade Biologics) medium including a LSGS (Low Serum Growth Supplement) kit in an incubator under the condition of 37 °C and 5% CO₂ for 24 hours. The *Youngia denticulata* extract of Example 1 was dissolved in water at a concentration of 1% to form a concentrate, and BaP, cadmium (Cd), lead (Pb) and nickel (Ni) were dissolved in DMSO at a concentration of 1 mM to prepare a concentrate. Thereafter, each concentrate was diluted with a medium and added to each well to be treated, and then cultured for 60 hours, and after completing the culturing, the medium was removed, and then 10 µl of 3-(4,5-dimethylthiazol-2-yl)-2,5-diphenyltetrazolium bromide (MTT) dissolved in PBS at 5 mg/ml was added, respectively, and the reaction was carried out under the condition of 37 °C and 5% CO₂ for 3 hours. Thereafter, the supernatant was carefully removed, and MTT formazan precipitated in cells was dissolved in 100 µl of DMSO, and then absorbance at 570 nm was measured using an ELISA reader (FIGS. 1, 2A, 2B and 2C).

As a result, in the case of HDFn cells treated with 0.005% or 0.01% *Youngia denticulata* extract, it was confirmed that decrease in cell viability by cytotoxicity and genetic material damage caused by 1µM and 2µM BaP was significantly low, and decrease in cell viability by treatment with 20 µM cadmium (Cd), 30 uM cadmium (Cd), 5 µM lead (Pb), 10 uM lead (Pb), 50 µM nickel (Ni) and 100 µM nickel (Ni) was recovered after treatment with *Youngia denticulata* extract.

### [Example 3]

### Measurement of anti-inflammatory effect when treating damaged cells by BaP with Youngia denticulata extract

It was analyzed with RT-PCR whether the *Youngia denticulata* extract of Example 1 decreases the increase in inflammatory cytokine IL-6 (Interleukin 6) and IL-8 (Interleukin 8) by BaP treatment to provide an anti-inflammatory effect.

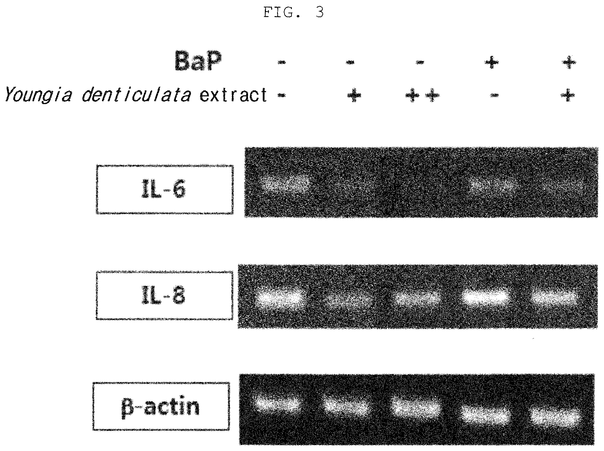

As the specific experimental method, HEKn cells (Human Epidermal Keratinocytes, neonatal), human-derived epidermal keratinocytes were constantly divided into 1 x 10⁵ cells on a 6 well plate for culturing, and cultured in an EpiLife medium (Gibco Cascade Biologics) including 1X HKGS (Human Keratinocyte Growth Supplement) and 1X Antibiotics in an incubator under 37 °C and 5% CO₂ for 24 hours. The *Youngia denticulata* extract of Example 1 was dissolved in water at a concentration of 1% to form a concentrate, and BaP was dissolved in DMSO at a concentration of 1 mM to prepare a concentrate. After completing the culturing, the medium was removed and the medium was changed to a medium without HKGS, and then each concentrate was diluted with the medium and treated by being added to each well, and then cultured for 24 hours. After completing the culturing, the cells were disrupted with Trizol (Ambion), the whole mRNA was collected with chloroform/isopropanol, then cDNA was synthesized by reverse transcriptase, and RT-PCR (Thermal Cycler, Bio-Rad) was performed using a specific primer to IL-6 and IL-8. After performing PCR, analysis was performed with agarose gel electrophoresis, and the result is shown in FIG. 3.

As a result, the HEKn cells treated with 0.005% or 0.01% *Youngia denticulata* extract decreased expression of IL-6 and IL-8 genes which were increased by 2µM BaP. Therefore, it was confirmed that the *Youngia denticulata* extract has an anti-inflammatory effect of protecting cells by decreasing the cell inflammation factor from environmental pollution by BaP.

### [Preparation Example 1]

40 mg of *Youngia denticulata* extract dry powder of Example 1
30 mg of stearic acid
30 mg of cetyl alcohol
10 mg of glyceryl monostearate
5 mg of glyceryl stearate
40 mg of glycerin
50 mg of butylene glycol
50 mg of wax
50 mg of liquid paraffin

An adequate amount of purified water was mixed with stirring, and heated to 80 °C to be emulsified in an emulsification reactor, and if required, an adequate amount of further additive was used. Thereafter, when emulsification was completed, cooling was carried out at room temperature.

### [Preparation Example 2]

300mg of *Youngia denticulata* extract dry powder of Example 1
30 mg of sugar
5 g of fructose
5 g of glucose
75 mg of sodium carboxymethylcellulose

Purified water was added to prepare a total of 50 ml of an additive which may be added to food.

### [Preparation Example 3]

15mg of *Youngia denticulata* extract dry powder of Example 1
15 mg of starch powder
25 mg of lactose
2 mg of talc
An adequate amount of magnesium stearate

The above components were mixed to prepare a tablet according to a preparation method of a tablet commonly known in the art.

The *Youngia denticulata* extract of the present invention is effective for prevention and treatment of damaged cell, induced cytotoxicity, damaged genetic material by a polycyclic aromatic hydrocarbon-based environmental pollution substance and heavy metals. Further, it may be used for treatment and prevention of diseases caused by a polycyclic aromatic hydrocarbon-based environmental pollution substance and heavy metals.

## Claims

1. A health functional food for prevention or treatment of diseases caused by a polycyclic aromatic hydrocarbon-based environmental pollution substance, comprising *Youngia denticulata* extract as an effective component.

2. An anti-inflammatory health functional food against inflammation caused by a polycyclic aromatic hydrocarbon-based environmental pollution substance, comprising *Youngia denticulata* extract as an effective component.

3. An anti-inflammatory pharmaceutical composition against inflammation caused by a polycyclic aromatic hydrocarbon-based environmental pollution substance, comprising *Youngia denticulata* extract as an effective component.

4. An anti-inflammatory cosmetic composition against inflammation caused by a polycyclic aromatic hydrocarbon-based environmental pollution substance, comprising *Youngia denticulata* extract as an effective component.

5. A composition for prevention or treatment of cell damage caused by a polycyclic aromatic hydrocarbon-based environmental pollution substance, comprising *Youngia denticulata* extract as an effective component.

6. A composition for prevention or treatment of cell damage caused by heavy metals, comprising *Youngia denticulata* extract as an effective component.

7. A food composition for releasing heavy metals, comprising *Youngia denticulata* extract as an effective component.

8. A food composition for detoxifying heavy metals, comprising *Youngia denticulata* extract as an effective component.
